# EUROPEAN PATENT SPECIFICATION

(11) **EP 2 285 218 B1**
(45) Date of publication and mention of the grant of the patent: **17.06.2020**
(21) Application number: 09734637.3
(22) Date of filing: 24.04.2009
(51) Int. Cl.: A23L 33/175, A61K 31/198, A61K 31/405, A61K 31/4172, A61K 45/06, A61P 39/00

(54) **DIETARY COMPOSITIONS AND METHODS FOR PROTECTION AGAINST CHEMOTHERAPY OR RADIOTHERAPY**
DIÄTPRODUKTZUSAMMENSETZUNGEN UND VERFAHREN ZUM SCHUTZ GEGEN CHEMOTHERAPIE ODER RADIOTHERAPIE
COMPOSITIONS ALIMENTAIRES ET PROCÉDÉS POUR LA PROTECTION CONTRE LA CHIMIOTHÉRAPIE OU LA RADIOTHÉRAPIE

(30) Priority: 24.04.2008 US 47680 P
(43) Date of publication of application: 23.02.2011
(73) Proprietor: University Of Southern California, USC Stevens, Los Angeles, CA 90089-2561 (US)
(72) Inventor: LONGO, Valter, Los Angeles, CA 90089-2561 (US)
(74) Representative: GPI Brevets
(86) International application number: PCT/US2009/041736
(87) International publication number: WO 2009/132320

(56) References cited:
- EP-A1- 0 232 652
- EP-A1- 0 560 989
- WO-A1-2008/123298
- GB-A- 2 029 220
- US-A- 5 292 723
- US-A1- 2006 275 506
- US-A1- 2007 009 576
- US-B1- 6 338 856
- , 25 September 2018 (2018-09-25), Retrieved from the Internet: URL:https://en.wikipedia.org/wiki/Oxidativ e_stress [retrieved on 2018-09-25]

## Description

### FUNDING

This invention was made with support in part by grants from the National Institutes of Health, AG20642, AG025135, GM075308, and Neurosciences Blueprint. Therefore, the U.S. government has certain rights.

### FIELD OF THE INVENTION

The present invention provides dietary compositions for use in a method for protecting animals or humans exposed to chemotherapy or radiotherapy.

### BACKGROUND OF THE INVENTION

Modern chemotherapy can improve the quality of life of cancer patients via palliation of cancer-related symptoms, and can significantly extend survival in many malignancies as well. However, the inevitable toxic side-effects frequently limit dose intensity and frequency of drugs administration. For instance, the use of doxorubicin or cisplatin can effectively treat many malignancies, but the drug-induced cardiotoxicity and nephrotoxicity, respectively, limit their full potential. Thus, reducing undesired toxicity by selectively protecting normal cells without compromising cancer targeting would prove beneficial to chemotherapy and enhance clinical outcome.

The document EP0560989 discloses an enteral preparation, which can be orally ingested, in lieu of parenteral amino acid infusion, to nourish cancer patients and inhibit growth of cancer cells. Enteral preparations disclosed in the document EP0560989 do not contain Methionine for treating cancer, resulting in a marked depression of methionine levels in cancer cells. In test example 2, the animals received an enteral preparation during 7 days and the treatment with the anticancer drug (5-FU) was given on days 1 to 6. The enteral preparation is thus given 24 hours after having received the anticancer drug or chemotherapy, such drug being potentiated in its activity by the administration of the enteral preparation. There is here a synergistically potentiation of the tumor growth inhibitory action of the anticancer drug.

The document GB2029220 discloses an amino acid solution for treating patient suffering from cancer. The amino acid solution, which does not contain Methionine, acts to reduce cancer cells or suppress cancers while maintaining the effect of giving nourishment of the amino acids contained in. In the two described experiments, there is potentiation of the tumor growth inhibitory action of the anticancer drug.

The document EP0232652 discloses nutritional compositions for inhibiting development of cancer and more particularly metastasis in mammal. The compositions are supplemented in Homocystein. Methionine may be present in the compositions. However, proteins are present in the compositions by the presence of soya protein in a total weight at the most of 1% on the total dry weight of constituents. In an example, soya proteins are present and are compared with control compositions (casein, powder milk) for the efficacy of the deficiency in Methionine content of the compositions.

The document EP2135604 describes a nutritional composition for cancer patients that comprises methionine in an amount of 0.1-5.0 g/100g and tryptophan in an amount of 0.02-3.0 g/100g, and L-isoleucin, L-leucine, L-lysine, L-phenylalanine, L-threonine and L-valine. A composition comprises 0.1 to 3.0 g/L L-methionine, 0.05 to 1.0 g/L L-tryptophan, and L-isoleucin, L-leucine, L-lysine, L-phenylalanine, L-threonine and L-valine. The document EP2135604 fails to disclose that the composition is administered for 3-10 days prior to chemotherapy treatment. Moreover, the document EP2135604 only describes peripheral administration by infusion.

### SUMMARY OF THE INVENTION

The present invention relates to dietary compositions for use in a method for protection against animals or humans exposed to chemotherapy or radiotherapy as defined in the claims. The method comprises administering a composition of the invention to an animal or human, thereby protecting the animal or human against chemotherapy or radiotherapy. The method may further comprise exposing the animal or human to the chemotherapy or radiotherapy. In some embodiments, the composition is administered to the animal or human for 3-10 consecutive days prior to the exposing step, 24 hours following the exposing step, or a combination thereof. In some embodiments, the composition is administered every third meal or every 3-10 days to protect the animal or human against aging.

The above-mentioned and other features of this invention and the manner of obtaining and using them will become more apparent, and will be best understood by reference to the following description, taken in conjunction with the accompanying drawings. The drawings depict only typical embodiments of the invention and do not therefore limit its scope.

### BRIEF DESCRIPTION OF THE FIGURES

Figure 1. Graph of (A) % survival, (B) methionine diet body weight %, (C) methionine fOOd intake, (D) post treatment body weight %, and (E) post treatment fOOd intake as a function of days. Mice were treated with a low methionine amino acid mix (LMAl) before treatment with doxorubicin.
Figure 2. Graph of (A) % survival, (B) post treatment body weight %, and (C) tryptophan diet body weight % as a fonction of days. Mice were treated with a low tryptophan amino acid mix (LTAl) before treatment with doxorubicin.

### DETAILED DESCRIPTION OF THE INVENTION

The present invention is based, at least in part, upon the unexpected discovery that dietary compositions comprising reduced level of methionine, tryptophan, ail amino acids, or protein, can be used to protect subjects against chemotherapy or radiotherapy.

More specifically, one dietary composition of the invention contains 0.05 - 0.2% (e.g., 0.02%, 0.05%, 0.1%, or 0.15%) by weight L-methionine and at least 0.05% (e.g., 0.1%, 0.5%, 1%, or 2%) by weight of each of L-tryptophan, L-isoleucine, L-leucine, L-lysine, L-phenylalanine, L-threonine, and L-valine, but no protein. In some embodiments, the composition also contains one or more amino acids selected from the group consisting of L-alanine, L-asparagine, L-aspartic acid, L-cysteine, L-glutamatic acid, L-glutamine, L-glycine, L-proline, L-serine, L-tyrosine, L-arginine, and L-histidine, e.g., each in the amount of at least 0.05% (e.g., 0.1%, 0.5%, 1%, or 2%) by weight. In some embodiments, the composition contains a normal amount of each of L-tryptophan, L-isoleucine, L-leucine, L-lysine, L-phenylalanine, L-threonine, L-valine, L-alanine, L-asparagine, L-aspartic acid, L-cysteine, L-glutamatic acid, L-glutamine, L-glycine, L-proline, L-serine, L-tyrosine, L-arginine, and L-histidine.

A second dietary composition of the invention contains 0-0.2% (e.g., 0.02%, 0.05%, 0.1%, or 0.15%) by weight L-tryptophan and at least 0.05% (e.g., 0.1%, 0.5%, 1%, or 2%) by weight of each of L-methionine, L· isoleucine, L-leucine, L-lysine, L-phenylalanine, L-threonine, L-valine, but no protein. In some embodiments, the composition also contains one or more amino acids selected from the group consisting of L-alanine, L- asparagine, L-aspartic acid, L-cysteine, L-glutamatic acid, L-glutamine, L-glycine, L-proline, L-serine, L-tyrosine, L-arginine, and L-histidine, e.g., each in the amount of at least 0.05% (e.g., 0.1%, 0.5%, 1%, or 2%) by weight. In some embodiments, the composition contains a normal amount of each of L-methionine, L-isoleucine, L-leucine, L-lysine, L-phenylalanine, L-threonine, L-valine, L-alanine, L-asparagine, L-aspartic acid, L-cysteine, L-glutamatic acid, L-glutamine, L-glycine, L-proline, L-serine, L-tyrosine, L-arginine, and L-histidine.

A third dietary composition of the invention contains L-methionine, L-tryptophan, L-isoleucine, L-leucine, L-lysine, L-phenylalanine, L-threonil!e, L-valine, L-alanine, L-asparagine, L-aspartic acid, L-cysteine, L-glutamatic acid, L-glutamine, L-glycine, L-proline, L-serine, L-tyrosine, L-arginine, and L-histidine, each in the amount of 0-0.2% (e.g., 0.02%, 0.05%, 0.1%, or 0.15%) by weight, but no protein.

A dietary composition of the invention can be used to protect an animal or human against chemotherapy or radiotherapy. More specifically, an animal or human may be fed with a dietary composition of the invention. When the animal or human is exposed to chemotherapy or radiotherapy normal cells, but not abnormal cells such as cancer cells, in the animal or human are protected. For example, the composition may be administered to the animal or human for 3-10 consecutive days prior to the animal or human is exposed to chemotherapy or radiotherapy. The composition may also be administered to the animal or human for 24 hours following the exposure. Preferably, the composition may be administered to the animal or human for bath 3-10 consecutive days prior to the animal or human is exposed to chemotherapy or radiotherapy and 24 hours following the exposure. For protection of an animal or human against aging, the composition may be administered every third meal or every 3-10 days.

Examples of chemotherapy include, but are not limited to, etoposide, doxorubicin, cisplatin, 5-FU, gemcitabine, cyclophosphamide, docetaxel, cyclophosphamide, carboplatin, GMZ, and paclitaxel. These drugs may be used individually or in combination.

The following examples are intended to illustrate the scope of the invention. While such examples are typical of those that might be used, other procedures known to those skilled in the art may alternatively be utΔzed. Indeed, those of ordinary skill in the art can readily envision and produce further embodiments, based on the teachings herein, without undue experimentation.

### EXAMPLE I

The strategies to treat cancer have focused largely on increasing the toxicity to tumor cells. The inventor has departed from the classic tumor-centric drug development focused on tumor killing and put focus on increasing the protection of normal cells. Recently, the inventor reported that a short-term starvation (STS; 40-60 hours) can enhance host resistance to chemotherapy while concomitantly enhancing tumor sensitivity to chemotherapy-induced apoptosis (Differential Stress Resistance, DSR) (1). The foundation of STS comes from the work of Dr. Longo in the aging field where growth-factor suppression and calorie restriction (CR) increase lifespan and stress resistance in various organisms. However, although a STS is a powerful method to differentially protect the host, it could have limited application in clinical settings. Therefore, the inventor investigated alternative pharmaceutical interventions that could also enhance hast resistance against chemotherapy. During the search, the inventor determined 3 promising preparations that provided increased protection to the host against chemotherapy drugs. The pharmaceutical preparations that were effective in enhancing resistance against chemotherapy were **1)** a methionine restricted amino acid mix (LAMl), 2) a tryptophan restricted amino acid mix (LTAl), and 3) glycerol (G1). LMA1 is effective only if the diet lacks other sources of methionine and LTA1 is effective only if the diet lacks other sources of tryptophan. Finally, G1 is effective in combination with a glucose-restricted diet. Interestingly, despite the fact that the diets were isocaloric and the fOOd intake was similar, LMAl/LTAl treated animals showed a lower weight profile. This suggests that LMAl/LTAl allow the animals to shift the energy towards 'maintenance' rather than 'growth/reproduction', and therefore increases resistance against chemotherapy toxicity.

### LMAl mix

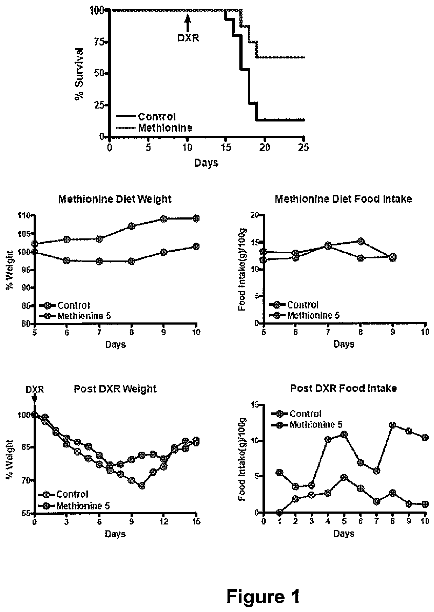

Methionine restriction has been shown to increase lifespan and stress resistance in laboratory rodents (2, 3). Therefore, the effect of a low methionine amino acid mix (LMAl) in the absence of proteins in the diet in protection against chemotherapy toxicity in laboratory rodents was investigated.5 days prior to chemotherapy, eight mice were given the LMAl mix in combination with a protein-free diet (Harlan, TD. 07789).
Methionine levels in the LMA1 mix were 20% of that of the control diet (TD. 07788). Following the 5-day LMA1 diet, mice were intravenously injected with a high-dose of doxorubicin (DXR, a widely used chemotherapy drug). To determine the degree of toxicity, mice were monitored daily for weight loss and abnormal behavior. Body weight and fOOd intake was recorded daily. LMA1 treated mice recovered from the weight loss more quickly compared to the control group (Fig. 1). Furthermore, LMAl-treated mice showed significantly higher survival rate compared to the control m1ce following high-dose chemotherapy (63% vs. 13% respectively) (Fig. 1).

### LTAl mix

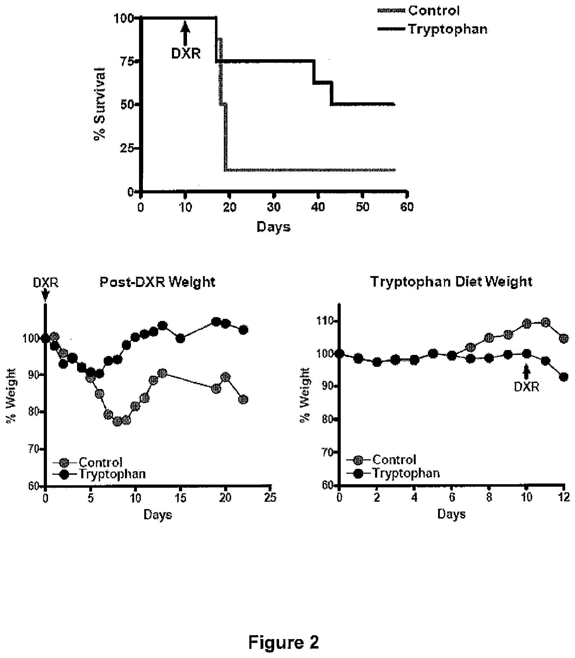

As with methionine restriction, a diet with low levels of tryptophan has also been shown to increase lifespan and decrease some age-related disease including cancer (4-7). Based on the fact that there is a strong correlation between longevity and stress resistance, the inventors believed that treatment of mice with a low tryptophan amino acid mix in the absence of other sources of tryptophan could also provide increased stress resistance in addition to lifespan extension. 10 days prier to chemotherapy,
eight mice were treated with the LTA1 mix in combination with a diet lacking protein (Harlan, TD. 07790). Tryptophan levels in the LTA1 mix was 20% of that of the control diet (TD. 07788). Toxicity was determined as clone with the LMA1 mix experiments. The LTA1 mix improved weight management after chemotherapy, causing a quicker recovering of the
weight loss compared to controls (Fig. 2). Also, mice treated with the LTAl mix had a 4-fold higher survival rate compared to the controls (50% vs 12.5%) (Fig. 2).

### MATERIALS AND METHODS

### LMA1 and LTAl

LMA1 and LTA1 are based on purified synthetic amino acid mixes (1) and were custom manufactured for us by Harlan Tekald in a 112" pellet form. All groups including Control (TD. 07788), LMA1 (TD. 07790), and LTA1 (TD. 07789) received isocaloric diets (3.9 KcaVg).

LMA1 mix: CD-1 mice, weighing 25-30 g, were prefed for 5 days prior to chemotherapy with purified synthetic amino acids mixes containing either normal (0.86%) or low (0.17%) levels of methionine.

LTA1 mix: CD-1 mice, weighing 25-30 g, were prefered for 5 days prior to chemotherapy with purified synthetic amino acids mixes containing
either normal (0.86%) or low (0.17%) levels of tryptophan.

**Table 1 . Composition of control diet**

| Formula | g/Kg |
|---|---|
| L-Alanine | 3.5 |
| L-Arginine HCl | 12.1 |
| L-Asparagine | 6.0 |
| L-Aspartic Acid | 3.5 |
| L-Cystine | 3.5 |
| L-Glutamic Acid | 40.0 |
| Glycine | 23.3 |
| L-Histidine HCI, monohydrate | 4.5 |
| L-Isoleucine | 8.2 |
| L-Leucine | 11.1 |
| L-Lysine HCl | 18.0 |
| L-Methionine | 8.6 |
| L-Phenylalanine | 7.5 |
| L-Proline | 3.5 |
| L-Serine | 3.5 |
| L-Threonine | 8.2 |
| L-Tryptophan | 1.8 |
| L-Tyrosine | 5.0 |
| Sucrase | 344.53 |
| Corn Starch | 150.0 |
| Maltodextrin | 150.0 |
| Soybean Oil | 80.0 |
| Cellulose | 30.0 |
| Mineral Mix, AIN-93M-MX (94049) | 35.0 |
| Calcium Phosphate, monobasic, monohydrate | 8.2 |
| Vitamin Mix, AIN-93-VX (94047) | 19.5 |
| Choline Bitartrate | 2.75 |
| TBHQ, antioxidant | 0.02 |

### Doxorubicin studies in mice

Following treatments with LMA1 or LTAl, mice were intravenously injected with 24-26 mg/kg doxorubicin (Bedford Laboratories) with 30 gauge insulin syringes (Becton, Dickinson and Company). Doxorubicin was dissolved in puri:fied water and diluted in saline to a final concentration of 5 mg/ml. All doxorubicin injections were followed by a saline/heparin wash to minimize endothelial cell damage. To determine toxicity and efficacy, mice were monitored routinely for weight loss and general behavior. Body
weight was recorded once daily throughout the experiment. Mice found moribund were sacrificed by C02 narcosis and necropsy was performed. Since cardiotoxicity is the major cause of death from acute doxorubicin toxicity, we prepared histological slides to examine the degree of damage at the tissue level.

### References

1. Raffaghello L, Lee C, Safdie FM, Wei M, Madia F, Bianchi G, & Longo VD (2008) Proc Natl Acad Sei USA.
2. Miller RA, Buehner G, Chang Y, Harper JM, Sigler R, & Smith-Wheelock M (2005) Aging Cell 4, 119-125.
3. Orentreich N, Matias JR, DeFelice A, & Zimmerman JA (1993) J Nutr 123, 269-274.
4. Ooka H, Segall PE, & Timiras PS (1988) Mech Ageing Dev 43, 79-98.
5. Segall PE & Timiras PS (1976) Mech Ageing Dev 5, 109-124.
6. Timiras PS, Hudson DB, & Segall PE (1984) Neurobiol Aging 5, 235-242.
7. Anisimov VN (2001) Exp Gerontol 36, 1101-1136.
8. Masoro EJ (2005) Mech Ageing Dev 126, 913-922.
9. Kennedy BK, Steffen KK, & Kaeberlein M (2007) Cell Mol Life Sei 64, 1323-1328.
10. Matsubara M, Yamagami K, Kitazawa Y, Kawamoto K, & Tanaka T (1996) Arch Toxicol 70, 514-518.
11. Migliaccio E, Giorgio M, Mele S, Pelicci G, Reboldi P, Pandolfi. PP, Lanfrancone L, & Pelicci PG (1999) Nature 402, 309-313.
12. Rogers QR & Harper AE (1965) JNutr 87, 267-273.

## Claims

1. A dietary composition for use in a method of protecting an animal or human against chemotherapy or radiotherapy, including administering the dietary composition comprising:
0.05-0.2% (by weight) L- methionine;
L-tryptophan, L-isoleucine, L-leucine, L-lysine, L-phenylalanine, L- threonine, and L-valine
in the amount of at least 0.05% (by weight) each;
and
no protein,
the composition being administered to an animal or human for 3-10 consecutive days prior the animal or human being exposed to chemotherapy or radiotherapy.

2. The dietary composition for the use of claim 1, further comprising one or more amino acids selected from the group consisting of L-alanine, L-asparagine, L- aspartic acid, L-cysteine, L-glutamatic acid, L-glutamine, L-glycine, L- proline, L-serine, L-tyrosine, L-arginine, and L-histidine.

3. The dietary composition for the use of claim 1 wherein the composition is further administered to the animal or human for 24 hours following the animal or human being exposed to chemotherapy or radiotherapy.

4. A dietary composition for use in a method of protecting an animal or human against chemotherapy or radiotherapy, including administering the dietary composition comprising:
0-0.2% (by weight) L- tryptophan; L-methionine, L-isoleucine, L-leucine, L-lysine, L-phenylalanine, L- threonine, and L-valine, in the amount of at least 0.05% (by weight) each; and
no protein,
the method comprising administering the composition to an animal or human for 3-10 consecutive days prior the animal or human being exposed to chemotherapy, or radiotherapy.

5. The dietary composition for the use according to claim 4 wherein the administered dietary composition further comprises one or more amino acids selected from the group consisting of L-alanine, L-asparagine, L-aspartic acid, L-cysteine, L-glutamatic acid, L-proline, L-serine, L-tyrosine, L-arginine, and Lhistidine.

6. The dietary composition for the use of claim 5 wherein the dietary composition is further administered to the animal or human for 24 hours following the animal or human is exposed to chemotherapy or radiotherapy.

## Patentansprüche

1. Diätetische Zusammensetzung zur Verwendung in einem Verfahren zum Schutz eines Tieres oder Menschen gegen Chemotherapie oder Strahlentherapie, welches die Verabreichung der diätetischen Zusammensetzung beinhaltet, umfassend:
0,05-0,2 % (nach Gewicht) L-Methionin;
L-Tryptophan, L-Isoleucin, L-Leucin, L-Lysin, L-Phenylalanin, L-Threonin und L-Valin in einer Menge von jeweils mindestens 0,05% (nach Gewicht); und
kein Protein,
wobei die Zusammensetzung einem Tier oder einem Menschen an 3-10 aufeinander folgenden Tagen verabreicht wird, bevor das Tier oder der Mensch einer Chemotherapie oder Strahlentherapie ausgesetzt wird.

2. Diätetische Zusammensetzung zur Verwendung nach Anspruch 1, die ferner eine oder mehrere Aminosäuren enthält, die aus der Gruppe ausgewählt sind, die aus L-Alanin, L-Asparagin, L-Asparaginsäure, L-Cystein, L-Glutaminsäure, L-Glutamin, L-Glycin, L-Prolin, L-Serin, L-Tyrosin, L-Arginin und L-Histidin besteht.

3. Diätetische Zusammensetzung zur Verwendung nach Anspruch 1, wobei die Zusammensetzung dem Tier oder dem Menschen 24 Stunden lang weiter verabreicht wird, nachdem das Tier oder der Mensch einer Chemotherapie oder Strahlentherapie ausgesetzt wurde.

4. Diätetische Zusammensetzung zur Verwendung in einem Verfahren zum Schutz eines Tieres oder Menschen gegen Chemotherapie oder Strahlentherapie, welches die Verabreichung einer diätetischen Zusammensetzung beinhaltet, umfassend:
0-0,2 Gew.-% L-Tryptophan; L-Methionin, L-Isoleucin, L-Leucin, L-Lysin, L-Phenylalanin, L-Threonin und L-Valin in einer Menge von jeweils mindestens 0,05 Gew.-%; und kein Protein,
wobei das Verfahren die Verabreichung der Zusammensetzung an ein Tier oder einen Menschen für 3-10 aufeinanderfolgende Tage umfasst, bevor das Tier oder der Mensch einer Chemotherapie oder Strahlentherapie ausgesetzt wird.

5. Diätetische Zusammensetzung zur Verwendung nach Anspruch 4, wobei die verabreichte diätetische Zusammensetzung ferner eine oder mehrere Aminosäuren umfasst, die aus der Gruppe ausgewählt sind, die aus L-Alanin, L-Asparagin, L-Asparaginsäure, L-Cystein, L-Glutaminsäure, L-Prolin, L-Serin, L-Tyrosin, L-Arginin und L-Histidin besteht.

6. Diätetische Zusammensetzung zur Verwendung nach Anspruch 5, wobei die diätetische Zusammensetzung dem Tier oder Menschen 24 Stunden lang weiter verabreicht wird, nachdem das Tier oder der Mensch einer Chemotherapie oder Strahlentherapie ausgesetzt wurde.

## Revendications

1. Composition alimentaire pour une utilisation dans une méthode de protection d'un animal ou d'un être humain contre les effets d'une chimiothérapie ou d'une radiothérapie, incluant l'administration de la composition alimentaire qui comprend :
0,05 - 0,2 % (en poids) de L- méthionine ;
du L-tryptophane, de la L-isoleucine, de la L-leucine, de la L-lysine, de la L-phénylalanine, de la L- thréonine, et de la L-valine
dans une proportion d'au moins 0,05 % (en poids) de chaque ;
et
aucune protéine,
la composition étant administrée à un animal ou à un être humain pendant 3 à 10 jours consécutifs avant le traitement de l'animal ou de l'être humain par chimiothérapie ou radiothérapie.

2. Composition alimentaire pour une utilisation selon la revendication 1, comprenant en outre un ou plusieurs acides aminés choisis dans un groupe composé de L-alanine, de L-asparagine, d'acide L-aspartique, de L-cystéine, d'acide L-glutamique, de L-glutamine, de L-glycine, de L- proline, de L-serine, de L-tyrosine, de L-arginine, et de L-histidine.

3. Composition alimentaire pour une utilisation selon la revendication 1, dans laquelle la composition est en outre administrée à l'animal ou à l'être humain pendant 24 heures suivant le traitement de l'animal ou de l'être humain par chimiothérapie ou radiothérapie.

4. Composition alimentaire pour une utilisation dans une méthode de protection d'un animal ou d'un être humain contre les effets d'une chimiothérapie ou d'une radiothérapie, incluant l'administration d'une composition alimentaire qui comprend :
0 - 0,2 % (en poids) de L- tryptophane; de L-méthionine, de L-isoleucine, de L-leucine, de L-lysine, de L-phénylalanine, de L- thréonine, et de L-valine, dans la proportion d'au moins 0,05 % (en poids) de chaque ; et
aucune protéine,
la méthode comprenant l'administration de la composition à un animal ou à un être humain pendant 3 à 10 jours consécutifs avant le traitement de l'animal ou de l'être humain par chimiothérapie ou radiothérapie.

5. Composition alimentaire pour une utilisation selon la revendication 4, dans laquelle la composition alimentaire administrée comprend en outre un ou plusieurs acides aminés choisis dans un groupe composé de L-alanine, de L-asparagine, d'acide L-aspartique, de L-cystéine, d'acide L-glutamique, de L-proline, de L-sérine, de L-tyrosine, de L-arginine, et de L-histidine.

6. Composition alimentaire pour une utilisation selon la revendication 5, dans laquelle la composition alimentaire est en outre administrée à l'animal ou à l'être humain pendant 24 heures suivant le traitement de l'animal ou de l'être humain par chimiothérapie ou radiothérapie.
